# EUROPEAN PATENT APPLICATION

(11) **EP 1 118 611 A1**
(43) Date of publication of application: **25.07.2001**
(21) Application number: 99944807.9
(22) Date of filing: 24.09.1999
(51) Int. Cl.: C07D 263/22

(54) **PROCESS FOR THE PREPARATION OF OXAZOLIDINONE DERIVATIVE**

(30) Priority: 01.10.1998 JP 27983598; 09.12.1998 JP 35017598; 11.03.1999 JP 6517599
(71) Applicant: NIPPON CHEMIPHAR CO., LTD., Tokyo 101-8678 (JP)
(72) Inventor: YAMAMOTO, Masao, Misato-shi Saitama 341-0018 (JP); YOSHIDA, Shinichi, Funabashi-shi Chiba 273-0031 (JP)
(74) Representative: Albrecht, Thomas, Dr.
(86) International application number: JP9905214
(87) International publication number: WO0020403

(57) **Abstract**

The invention relates to a process for preparing (5RS)-5-benzyl-3-[(1SR)-3-morpholino-1-phenylpropyl]-1,3-oxazolidin-2-one or its pharmacologically acceptable salt which is of value as a remedy for treatment of incontinence of urine and thamuria and which is performed by the steps of:
dissolving an acid and a mixture of (2RS)-1- [(1SR)-3-morpholino-1-phenylpropyl]amino-3-phenyl-2-propanol and (2RS) -1- ((lRS,) -3-morpholino-1-phenylpropyl] amino-3-phenyl-2-propanol in a solvent to obtain a salt of the former (1SR)-compound utilizing difference of solubility between the salt of (1SR)-compound and a salt of the latter (1RS)-compound;
bringing a basic compound into contact with the salt of (1SR)-compound to produce a free (1SR)-compound;
reacting thus produced (2RS)-1-[(1SR)-3-morpholino-1-phenylpropyl]amino-3-phenyl-2-propanol with a compound having the formula (I):

   R₂C=O (I)

   wherein R represents a chlorine atom, an alkoxy group, an aryloxy group or an amino group, or a chloroformic acid ester; and
cyclizing the resulting product.

## Description

### [FIELD OF THE INVENTION]

The present invention relates to a process forpreparing an oxazolidinone derivative, and specifically to a process for preparing (5RS)-5-benzyl-3-[(1SR)-3-morpholino-1-phenylpropyl)-1,3-oxazolidin-2-one.

### [BACKGROUND OF THE INVENTION]

It is known that (5RS)-5-benzyl-3-[(1SR)-3-morpholino-1-phenylpropyl]-1,3-oxazolidin-2-one or a pharmacologically acceptable salt thereof shows a novel ability to relieve urinary contraction and hence is of value as a remedy for treatment of incontinence of urine and frequent micturition; EUR. J. PHARMACOL., 332(1), 89-95 (1997) and Japanese Patent Provisional Publication No. 6-80645.

The 5-benzyl-3-[3-morpholino-1-phenylpropyl]-1,3-oxazolidin-2-one contains two asymmetric carbon atoms and accordingly there are present a pair of diastereoisomers, namely, diastereoisomer A: (5RS)-5-benzyl-3-[(1SR)-3-morpholino-1-phenylpropyl]-1,3 -oxazolidin-2-one, and diastereoisomer B: (5RS)-5-benzyl-3-[(1RS)-3-morpholino-1-phenylpropyl]-1,3-oxazolidin-2-one. Both diastereoisomers are produced in approximately equal amounts when the oxazolidine derivative is synthesized in a conventional synthetic method.

It is generally difficult to convert the diastereoisomer B into the desired diastereoisomer A, and hence the diastereoisomer B is discarded. Therefore, it is desirable from the viewpoint of industrial production that division of diastereoisomers at an early stage of synthesis is conducted.

In the aforementioned Japanese Patent Provisional Publication No. 6-80645, the diastereoisomer A is obtained by the steps of reacting 5-benzyl-1,3-oxazolidin-2-one with 4-[(3-chloro-3-phenyl)propyl]morpholine hydrochloride and processing by column chromatography the resulting mixture of the diastereoisomer A and diastereoisomer B to separate the diastereoisomer A from the diastereoisomer B. In Fig. 1 of the above-mentioned Provisional Publication, a variety of processes for synthesizing 5-benzyl-3-(3-morpholino-1-phenylpropyl)-1,3-oxazolidin-2-one are described. However, there are no descriptions to indicate the separation of diastereoisomers at an early stage of the synthesis procedures.

In Japanese Patent Provisional Publication No. 7-247275, the diastereoisomer A is obtained according to the following synthetic route:

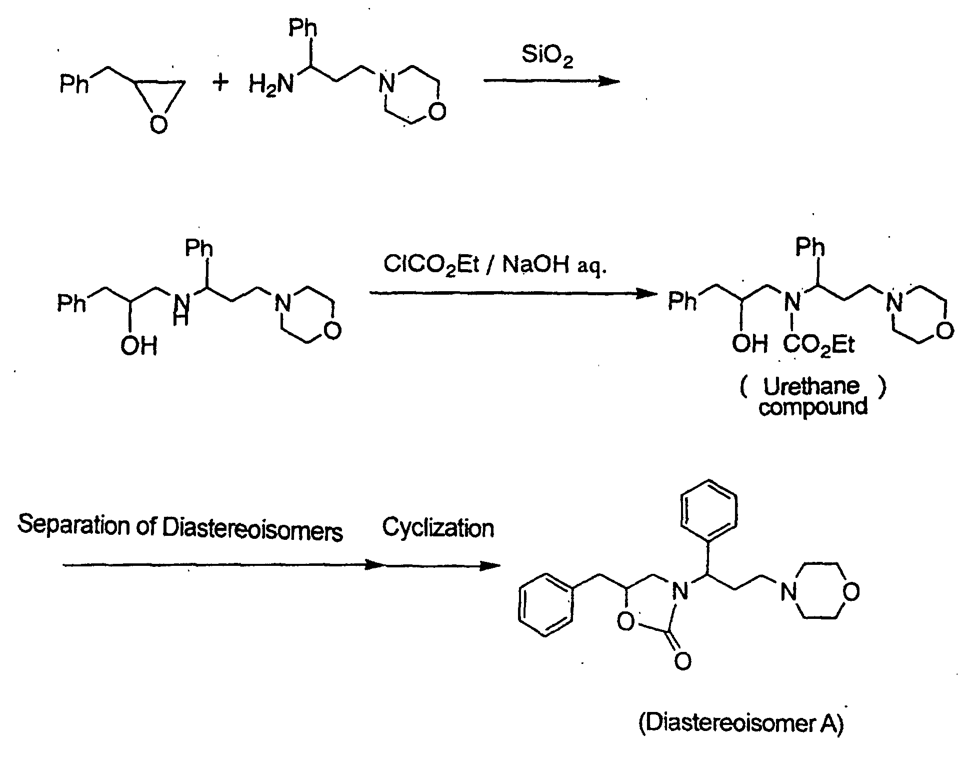

The above-illustrated route can be described as follows: 1-(3-morpholino-1-phenylpropyl)amino-3-phenyl-2-propanol is reacted with ethyl chloroformate; the resulting 1-[N-(3-morpholino-1-phenylpropyl)-N-ethoxy-carbonyl]amino-3-phenyl-2-propanol (i.e., urethane compound) is then treated in its fumaric acid salt form to separate the diastereoisomers; and one diastereoisomer is cyclized to give (5RS)-5-benzyl-3-[(1SR)-3-morpholino-1-phenylpropyl]-1,3-oxazolidin-2-one (diastereoisomer A).

### [DISCLOSURE OF THE INVENTION]

It is desired to provide a new process for preparing (5RS)-5-benzyl-3-[(1SR)-3-morpholino-1-phenylpropyl]-1,3-oxazolidin-2-one, particularly a synthetic process in which diastereoisomer separation is conducted at an early stage.

Based on the synthetic process described in the aforementioned Japanese Patent Provisional Publication No. 7-247275, the present inventors have discovered that (5RS) -5-benzyl-3- [(1SR) -3-morpholino-1-phenylpropyl] -1,3-oxazolidin-2-one is efficiently obtained by the steps of, first, separating diastereoisomers of 1-(3-morpholino-1-phenylpropyl)amino-3-phenyl-2-propanol containing two asymmetric carbon atoms and then cyclizing the resulting (2RS)-1-[(1SR) -3-morpholino-1-phenylpropyl] amino-3-phenyl-2-propanol. The present invention is based on the above-mentioned discovery.

### [Process-1]

The present invention relates to a process for preparing (5RS)-5-benzyl-3-[(1SR)-3-morpholino-1-phenylpropyl]-1,3-oxazolidin-2-one which comprises the steps of:
reacting (2RS) -1- [(1SR)-3-morpholino-1-phenyl-propyl]amino-3-phenyl-2-propanol with a compound having the formula. (I):

   R₂C=O (I)

   wherein R represents a chlorine atom, an alkoxy group, an aryloxy group or an amino group, or a chloroformic acid ester; and
cyclizing the resulting product.

### [Process-2]

The invention further relates to a process for preparing (5RS)-5-benzyl-3-[(1SR)-3-morpholino-1-phenyl-propyl]-1,3-oxazolidin-2-one which comprises the steps of:
dissolving an acid and a mixture of (2RS)-1-[(1SR)-3-morpholino-1-phenylpropyl]amino-3-phenyl-2-propanol and (2RS)-1-[(1RS)-3-morpholino-1-phenylpropyl]amino-3-phenyl-2-propanol in a solvent to obtain a salt of the former (1SR)-compound utilizing difference of solubility between the salt of (1SR)-compound and a salt of the latter (1RS)-compound;
bringing a basic compound into contact the salt of (1SR)-compound to produce a free (1SR)-compound;
reacting thus produced (2RS) -1-[(1SR)-3-morpholino-1-phenylpropyl]amino-3-phenyl-2-propanol with a compound having the formula (I) :

   R₂C=O (I)

   wherein R represents a chlorine atom, an alkoxy group, an aryloxy group or an amino group, or a chloroformic acid ester; and
cyclizing the resulting product.

### [PREFERRED EMBODIMENTS OF THE INVENTION]

### [Process-1]

The process-1 is further described in more detail.

In the compound of the formula (I), R can be a chlorine atom, an alkoxy group having 1 to 6 carbon atoms such as methoxy, ethoxy, or 2-chloroethoxy; an aryloxy group such as phenyloxy, 2-methoxyphenyloxy, 4-nitrophenyloxy, 2-pyridyloxy, or 1-imidazolyloxy; or an amino group. Preferred are methoxy, ethoxy, and phenyloxy. Most preferred is phenyloxy.

The chloroformic acid ester can have an alkyl moiety containing 1 to 6 carbon atoms. Examples of the esters include trichloromethyl chloroformate (TCF) and ethyl chloroformate.

The reaction between (2RS)-1-[(1SR)-3-morpholino-1-phenylpropyl]amino-3-phenyl-2-propanol (referred to as MAPP-A) and a compound of the formula (I) or the chloroformate can be performed in an inert solvent such as benzene or toluene in the presence of a base such as sodium hydroxide, ammonia, sodium methoxide, sodium ethoxide, potassium t-butoxide, or sodium hydride. The reaction can be performed at a temperature of 80 to 130°C, preferably at a reflux temperature of the solvent.

The reaction can be performed using one mol. of MAPP-A and 1 to 30 mol. of the compound of the formula (I) or chloroformate. If dimethyl carbonate or diethyl carbonate is employed in an excessive amount, as compared with the amount of MAPP-A, the carbonate can be utilized as the solvent. In the case that diphenyl carbonate is used, unreacted diphenyl carbonate can be converted into 1,3-oxazolidin-2-one by adding monoethanolamine after the reaction is complete and further refluxing the mixture, so that the desired product can be easily recovered.

Otherwise, MAPP-A is treated with an alkyl chloroformate, and the resulting urethane compound is isolated. The isolated urethane compound is subjected to cyclization reaction to obtain (5RS)-5-benzyl-3-[(1SR)-3-morpholino-1-phenylpropyl]-1,3-oxazolidin-2-one.

MAPP-A can be produced by the process described hereinbelow in the steps (1) to (3) of process-2.

### [Process-2]

The process-2 is described below.
(1) A mixture of (2RS)-1-[(TSR)-3-morpholino-1-phenylpropyl]amino-3-phenyl-2-propanol (MAPP-A) and (2RS)-1-[(1RS)-3-morpholino-1-phenylpropyl]amino-3-phenyl-2-propanol (MAPP-B) and an acid are dissolved in an organic solvent such as isopropanol or ethyl acetate, and the mixture is warmed until a homogeneous solution is produced.
   The acid preferably is an organic acid. More preferred are benzoic acid, succinic acid and p-toluenesulfonic acid. The acid is preferably used in an amount of 0.5 to 2 molar equivalents, per one molar equivalent of the free compound.
(2) The solution is then cooled to precipitate a crystalline product. The precipitation can be efficiently performed by a conventional method such as addition of seed crystals.
(3) The resulting salt of MAPP-A and acid is treated with a basic compound such as sodium hydroxide in a solvent to give a free compound.
(4) Thus obtained MAPP-A is processed in the manner described for Process-1, to give (5RS)-5-benzyl-3-[(1SR)-3-morpholino-1-phenylpropyl]-1,3-oxazolidin-2-one.

In the process of the invention, the mixture of MAPP-A and MAPP-B (i.e., starting compound mixture) can be obtained by any of the following synthesis methods:

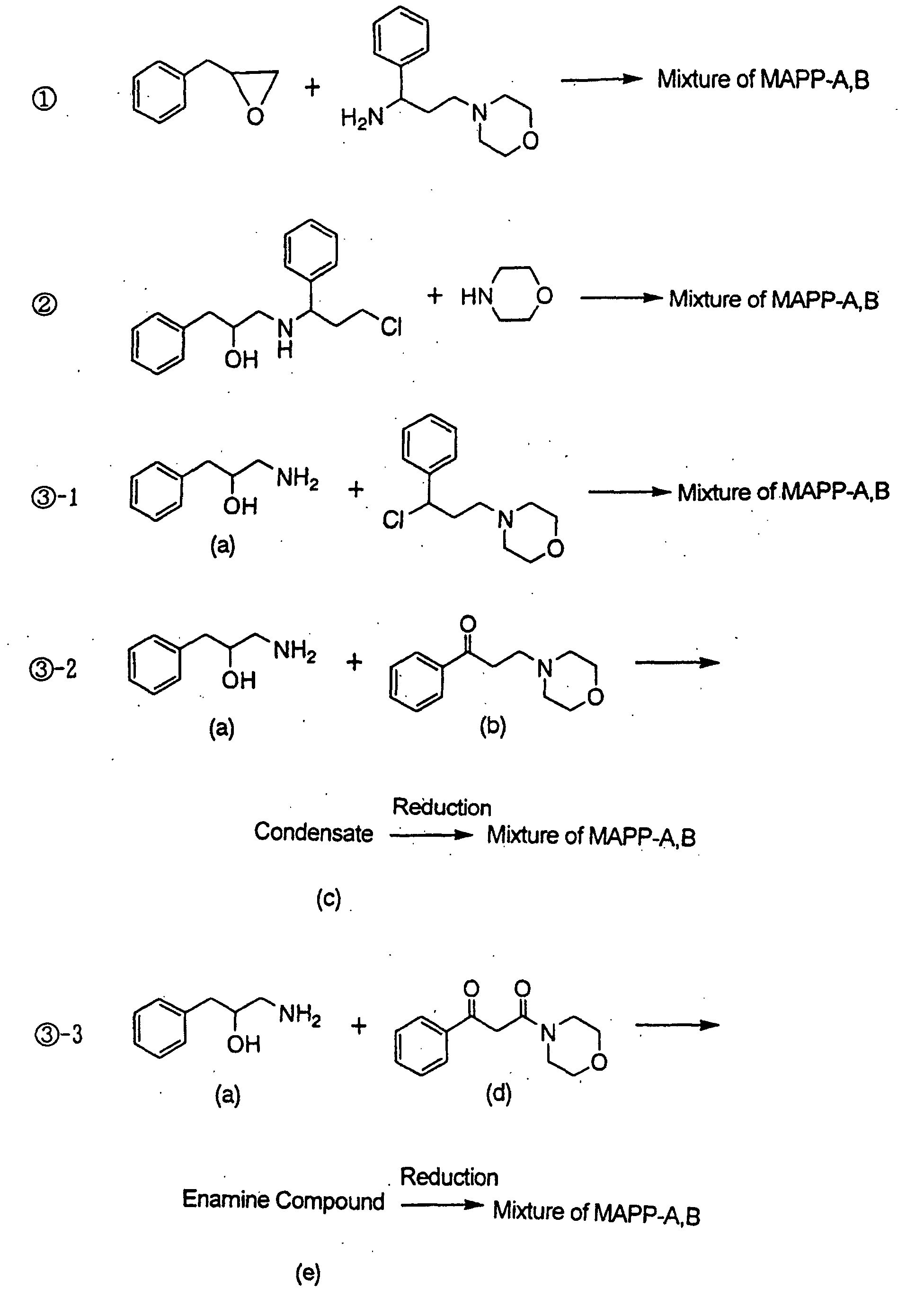

The procedures of the process (1) are described in Japanese Patent Provisional Publication No. 7-247275. The synthetic routes of the process (2) and process (3)-1 are described in Fig. 1 of Japanese Patent Provisional Publication No. 6-80645.

In the process (3)-2, 1-amino-3-phenyl-2-propanol of the formula (a) and the compound (b) are reacted, and the resulting condensation product (c) is reduced to give a mixture of MAPP-A and MAPP-B.

In the process (3)-3, 1-amino-3-phenyl-2-propanol of the formula (a) and the compound (d) are reacted, and the resulting enamine compound (e) is reduced to give a mixture of MAPP-A and MAPP-B.

In the processes (3)-2 and (3)-3, the reducing agent can be chosen to increase the yield of MAPP-A as compared with MAPP-B.

The (5RS)-5-benzyl-3- [(1SR) -3-morphclino-1-phenyl-propyl]-1,3-oxazolidin-2-one obtained in the process-1 or process-2 is treated with an organic acid (e.g., fumaric acid, tartaric acid, or hydrochloric acid), to obtain a salt of (5RS)-5-benzyl-3-[(1SR)-3-morpholino-1-phenyl-propyl]-1,3-oxazolidin-2-one with an organic acid.

MAPP-B which is a by-product produced in the process-2 can be converted into a mixture of MAPP-A and MAPP-B according to the following synthetic route:

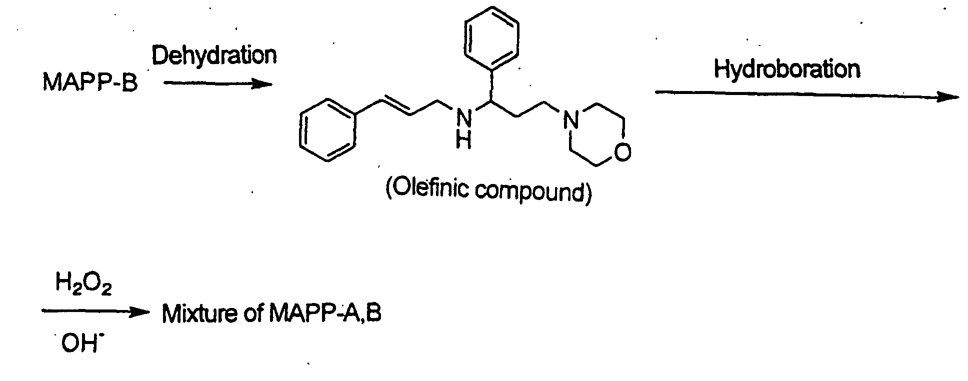

The reaction of from MAPP-B to its olefinic compound can be performed under heating in the presence of a base (e.g., sodium methoxide). The resulting olefinic compound is reacted with borane (BH₃) in a solvent such as THF, and subsequently the resulting product is reacted with hydrogen peroxide in the presence of a base (e.g., sodium hydroxide) to give a mixture of MAPP-A and MAPP-B.

### [Examples]

The present invention is described by the following reference examples and working examples.

### Process for preparation of a MAPP-A/MAPP-B mixture

### [Reference example 1]

1-(3-Morpholino-1-phenylpropyl)amino-3-phenyl-2-propanol
(a) In dry benzene (30 mL) were dissolved 454 mg (3.0 mM) of 1-amino-3-phenyl-2-propanol and 658 mg (3.0 mM) of 1-phenyl-3-morpholino-1-propanone. The resulting solution was reflxed under heating for 24 hours using a Dean-Stark trap. The solvent was distilled off under reduced pressure to give 1.06 g of a condensation product as a pale yellow oil.
(b) The crude condensation product (353 mg) obtained above was dissolved in dry methanol (10 mL) in a nitrogen gas atmosphere. To the solution was added 530 mg (2.50 mM) of sodium triacetoxyborohydride, under chilling conditions. The chilled mixture was stirred for 18 hours at room temperature. The solvent was distilled off under reduced pressure, and ether was added to the residue. The ether portion was washed successively with saturated aqueous sodium hydrogen carbonate solution, water, and saturated aqueous sodium chloride solution, and then dried over anhydrous sodium sulfate. The solvent was distilled off under reduced pressure to give 284 mg (MAPP-A/MAPP-B = 61/39) of the desired product as a pale yellow oil.

The ratio of diastereoisomer A/B was determined by HPLC under the following conditions:
Column: Nakarai Tesque COSMOSIL 5C18-AR (4.6x250 mm)
Eluent: 2% NaH₂PO₄/CH₃CN = 10/3
Detection: UV 215 nm
Flow rate: 1.0 mL/min.

### [Reference example 2]

### 1-(3-Morpholino-1-phenylpropyl)amino-3-phenyl-2-propanol

In the same manner as in Reference Example 1, the condensation product was reduced using triacetoxyborohydride tetramethylammonium (reducing agent) and dry ethanol (solvent), to give a crude product of the desired compound (MAPP-A/MAPP = 70/30). HPLC conditions were the same as those of Reference example 1.

### [Reference example 3]

### 1-(3-Morpholino-1-phenylpropyl)amino-3-phenyl-2-propanol

The crude product (1.06 g) of the condensation product which was prepared in the procedure of Reference example 1-(a) was dissolved in dry tetrahydrofuran (THF, 15 mL) in a nitrogen gas atmosphere. To the solution was added 3.6 mL (3.6 mM) of a triethylboran/THF solution, and the mixture was stirred for 30 minutes at room temperature. To the mixture was then added 137 mg (3.6 mM) of sodium borohydride, and the mixture was then stirred for 3 hours at room temperature. The solvent was distilled off under reduced pressure, and to the resulting residue were added 20 mL of water and 30 mL of 3N hydrochloric acid under cooling with ice. The mixture was stirred for 10 min. The aqueous mixture was washed with ether and made basic by addition of a saturated aqueous sodium hydrogen carbonate solution. The basic solution was extracted with ethyl acetate. The ethyl acetate extract was washed with water and a saturated aqueous sodium chloride solution and dried over anhydrous sodium sulfate. The solvent was distilled off under reduced pressure. The resulting crude residue was purified by medium liquid chromatography (chloroform - methanol/chloroform=1/5), to give 323 mg (yield: 31%, MAPP-A/MAPP-B = 69/31) of the desired product. The HPLC conditions were the same as those in Reference example 1.

### [Reference example 4]

### 1-(3-Morpholinocarbonyl-1-phenylpropenyl)amino-3-phenyl-2-propanol

In benzene (30 mL) were dissolved 4.54 g (30 mM) of 1-amino-3-phenyl-2-propanol, 7.00 g (30 mM) of N-(3-phenyl-1,3-dioxopropyl)morpholine, and 0.03 mL of methanesulfonic acid. The solution was heated under reflux for 24 hours using a Dean-Stark trap. The solvent was distilled off under reduced pressure. The resulting crude product was purified by medium liquid chromatography (methanol/chloroform = 1/50) to give 10.3 g (yield: 91%) of the desired product as a yellow oil.
¹H NMR (CDCl₃, 400 MHz) δ: 2.50 (1H, d, J=4Hz), 2.63-2.71 (2H, m), 3.01-3.17 (2H, m), 3.52-3.66 (8H, m), 3.84 (1H, m), 4.75 (1H, s), 7.11-7.38 (10H, m), 9.56 (1H, bs).

### [Reference example 5]

### 1-(3-Morpholino-1-phenylpropyl)amino-3-phenyl-2propanol

In dry THF (40 mL) was dissolved 1.53 g (5.0 mM) of N-tosyl-D,L-phenylglycine in a nitrogen gas atmosphere. To the solution was added 5.1 mL (5.0 mM) of 0.98 M/L of BH₃·THF complex under chilling with ice. The mixture was stirred for one hour at room temperature. Under chilling with ice, 1.69 g (4.43 mM) of 1-(3-morpholinocarbonyl-1-phenylpropenyl)amino-3-phenyl-2-propanol in dry THF (15 mL) was added, and the resulting mixture was stirred for one hour at room temperature. Subsequently, to the reaction mixture was added 23 mL (22.5 mM) of 0.98 M/L BH₃·THF complex at -78°C, and the mixture was stirred at -78°C for 6 hours. Further, 9 mL (8.8 mM) of 0.98 M/L BH₃·THF complex was added, and the mixture was stirred for 18 hours at room temperature. The reaction mixture was chilled with ice, and heated at 80°C for 2 hours after addition of 20 mL of water and 100 mL of 6N hydrochloric acid. The reaction mixture was cooled, washed with ether, and made basic by addition of 6N aqueous sodium hydroxide solution under chilling with ice. The mixture was extracted with ether. The ether portion was washed with water and a saturated sodium chloride solution, and dried over anhydrous sodium sulfate. The solvent was distilled off under reduced pressure. The residual crude product was purified by medium pressure liquid chromatography (methanol/chloroform = 1/5 to 1/30), to give 1.05 g (yield: 67%, MAPP-A/MAPP-B = 63/37) of the desired product as a pale yellow oil. The HPLC conditions were the same as those of Reference example 1.

### [Reference example 6]

### 1-(3-Morpholino-1-phenylpropyl)amino-3-phenyl-2-propanol

The procedures of Reference example 5 were repeated using N-tosyl-D,L-valine as amino acid, to give the desired product (MAPP-A/MAPP-B = 61/39). The HPLC conditions were the same as those of Reference example 1.

### [Reference example 7]

### 1-(3-Morpholino-1-phenylpropyl)amino-3-phenyl-2-propanol

The procedures of Reference example 5 were repeated using N-tosyl-D,L-2-naphthylglycine as amino acid, to give the desired product (MAPP-A/MAPP-B = 63/37). The HPLC conditions were the same as those of Reference example 1.

### Preparation of salt of MAPP-A with an organic acid

### [Example 1]

Salt of (2RS) -1- [(1SR)-3-morpholino-1-phenylpropyl]amino-3-phenyl-2-propanol with an organic acid

### (1-1) In the case that the acid was succinic acid

A mixture of 1-(3-morpholino-1-phenylpropyl)amino-3-phenyl-2-propanol (MAPP-A/MAPP-B = 48/52, 2.03 g, 5.73 mmol.) was dissolved in a mixture of ethyl acetate (10 mL) and ethanol (0.5 mL). The solution was warmed after addition of succinic acid (0.60 g, 5.08 mmol.), until the solution became homogeneous. The warmed solution was gradually cooled to room temperature, and stirred over-night at room temperature after addition of seed crystals (1 mg). The precipitated crystalline product was collected by filtration, washed with ethyl acetate (2 mL x 2), and dried under reduced pressure for 5 hours at room temperature, to give 0.73 g (yield: 26.7%) of monosuccinate of MAPP-A as a white crystalline product. MAPP-A/MAPP-B = 93.5/6.5

The ratio of diastereoisomers was determined in Examples 1 and 2 by HPLC under the following conditions:
Column: Nakarai Tesque COSMOSIL 5C18-AR (4.6x250 mm)
Moving phase: 2% NaH₂PO₄/CH₃CN = 10/3
Detection: UV 215 nm
Flow rate: 1.0 mL/min.
mp: 127.2 - 129.4 °C
¹H NMR (CD₃OD, 400 MHz) δ: 1.87-1.93 (1H, m), 2.26-2.36 (1H, m), 2.41-2.72 (13H, m), 2.76-2.84 (1H, m), 3.67-3.78 (4H, m), 4.07-4.14 (1H, m), 4.19-4.24 (1H, m), 7.10-7.27 (6H, m), 7.32-7.43 (4H, m).

### (1-2) In the case that the acid was succinic acid

A mixture of MAPP-A and MAPP-B (MAPP-A/MAPP-B = 48/52, 1.82 g, 5.13 mmol.) was dissolved in isopropanol (6 mL). The solution was warmed after addition of succinic acid (0.60 g, 5.08 mmol.), until the solution became homogeneous. The warmed solution was gradually cooled to room temperature, and stirred overnight at room temperature after addition of seed crystals (1 mg). The precipitated crystalline product was collected by filtration, washed with isopropanol (1 mL), and dried under reduced pressure for 3 hours at room temperature, to give 0.73 g (yield: # 17.7%) of monosuccinate of MAPP-A as a white crystalline product. MAPP-A/MAPP-B = 88.4/11.6

### (2-1) In the case that the acid was p-toluenesulfonic acid monohydrate

A mixture of MAPP-A and MAPP-B (MAPP-A/MAPP-B = 48/52, 2.03 g, 5.73 mmol.) was dissolved in isopropanol (6 mL). The solution was warmed after addition of p-toluenesulfonic acid monohydrate (0.97 g, 5.10 mmol.), until the solution became homogeneous. The warmed solution was gradually cooled to room temperature, and stirred overnight at room temperature after addition of hexane (6 mL) and seed crystals (1 mg). The precipitated crystalline product was collected by filtration, washed with 1.5 mL of isopropanol/hexane mixture (1/2), and dried under reduced pressure for 5 hours at room temperature, to give 0.66 g (yield: 21.2%) of mono-p-toluene-sulfonate of MAPP-A as a white crystalline product. MAPP-A/MAPP-B = 98.0/2.0
¹H NMR (CD₃OD, 400 MHz) δ: 1.87-1.93 (1H, m), 2.32-2.87 (10H, m), 2.37 (3H, s), 3.67-3.79 (4H, m), 4.12-4.17 (1H, m), 4.33-4.37 (1H, m), 7.11-7.42 (12H, m), 7.69-7.71 (2H, m).

### (3-1) In the case that the acid was benzoic acid

A mixture of MAPP-A and MAPP-B (MAPP-A/MAPP-B = 48/52, 1.89 g, 5.33 mmol.) and benzoic acid (0.45 g, 3.68 mmol.) were placed in isopropanol (14 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 6 mL of isopropanol, and dried under reduced pressure for 3 hours at room temperature, to give 1.30 g of a white crystalline product. The obtained product (1.29 g, 2.71 mmol.) was recrystallized from isopropanol (9 mL), and the recrystallized product was dried under reduced pressure for 3 hours to give 1.06 g (yield: 42.0%) of monobenzoate of MAPP-A as a white crystalline product.
mp: 133.2 - 134.6°C
MAPP-A/MAPP-B = 87.4/12.6
¹H NMR (CD₃OD, 400 MHz) δ: 1.87-1.92 (1H, m), 2.26-2.84 (11H, m), 3.65-3.76 (4H, m), 4.07-4.21 (2H, m), 4.33-4.37 (1H, m), 7.12-7.45 (13H, m), 7.92-7.97 (2H, m).

### (3-2) In the case that the acid was benzoic acid

A mixture of MAPP-A and MAPP-B (MAPP-A/MAPP-B = 48/52, 1.89 g, 5.33 mmol.) and benzoic acid (0.45 g, 3.68 mmol.) were placed in isopropanol (23 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 6 mL of isopropanol, and dried under reduced pressure for 3 hours at room temperature, to give 1.17 g of a white crystalline product. The obtained product (1.16 g, 2.43 mmol.) was recrystallized from isopropanol (8.1 mL), and the recrystallized product was dried under reduced pressure for 3 hours to give 0.92 g (yield: 36.5%) of monobenzoate of MAPP-A as a white crystalline product.
mp: 133.6 - 135.2°C
MAPP-A/MAPP-B = 92.1/7.9

### (3-3) In the case that the acid was benzoic acid

A. mixture of MAPP-A and MAPP-B (MAPP-A/MAPP-B = 59/41, 1.00 g, 2.82 mmol.) and benzoic acid (0.69 g, 5.65 mmol.) were placed in isopropanol (20 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 10 mL of isopropanol, and dried under reduced pressure at 40°C for 6 hours, to give 1.87 g (yield: 64.7%) of monobenzoate of MAPP-A as a white crystalline product.
MAPP-A/MAPP-B = 84.4/15.6

### (3-4) In the case that the acid was benzoic acid

A mixture of MAPP-A and MAPP-B (MAPP-A/MAPP-B = 59/41, 50.0 g, 141 mmol.) and benzoic acid (12.0 g, 98.3 mmol.) were placed in isopropanol (620 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 300 mL of isopropanol, and dried under reduced pressure at 40°C for 6 hours, to give 39.1 g of a white crystalline product. The crystalline product was recrystallized from isopropanol (390 mL). The recrystallized product was dried at 40°C for 7 hours, to give 32.8 g (yield: 48.8% from the MAPP-A,B mixture, and 82.7% from MAPP-A of the MAPP-A,B mixture) of monobenzoate of MAPP-A as a white crystalline product.
mp: 136.2 - 139.8°C
MAPP-A/MAPP-B = 97.2/2.8

The product showed a ratio of MAPP-A/MAPP-B = 95.5/4.5 when the determination was performed by HPLC under the following conditions:
Column: GL SCIENCES INARTSIL ODS-3V (4.6x250 mm)
Moving phase: 2% NaH₂PO₄/CH₃CN = 10/3
Detection: UV 215 nm
Flow rate: 1.0 mL/min.

### Preparation of (5RS) -5-benzyl-3- [(1SR)-3-morpholino-1-phenylpropyl]-1,3-oxazolidin-2-one fumarate

### [Example 2]

### (5RS)-5-benzyl-3-[(1SR)-3-morpholino-1-phenyl-propyl] -1,3-oxazolidin-2-one fumarate

(A) MAPP-A monobenzoate (MAPP-A/MAPP-B = 97.2/2.8, 1.00 g, 2.10 mmol.) obtained in Example 1 (3-4) was suspended in 1M aqueous sodium hydroxide solution (5 mL), and the solution was vigorously stirred for 10 minutes at room temperature after addition of toluene (5 mL). The organic solvent portion was then collected. The collected organic portion was dropwise added to a separately prepared toluene (5 mL) solution of trichloromethyl chloroformate (TCF, 0.28 mL, 2.32 mmol.) for a period of 5 minutes under chilling with ice. The mixture was stirred for 30 minutes at room temperature, and further stirred for one hour at room temperature after addition of 14% aqueous ammonia solution (10 mL). The mixture was further stirred for 30 minutes at room temperature after addition of TCF (0.14 mL, 1.16 mmol.), and the organic portion was collected. The organic portion was washed with saturated aqueous sodium chloride solution (10 mL), and dried over anhydrous sodium sulfate. The solvent was distilled off under reduced pressure. The residue was stirred on an oil bath at 100°C (outer temperature) for 10 minutes, after addition of 50% ethanol (2.6 mL) and fumaric acid (0.24 g, 2.07 mmol.). The heater of the oil bath was switched off. The mixture was still stirred on the oil bath for gradual cooling. When the temperature of the oil bath reached 80°C, seed crystals (1 mg) were added. The mixture was further cooled overnight under stirring. The precipitated crystalline product was collected by filtration, washed with 50% ethanol (1 mL x 2), and dried under reduced pressure at 40°C for 6 hours, to give 0.69 g (yield: 66.2%) of the desired compound as a white crystalline product.
mp: 165.2 - 166.4 °C

(B) MAPP-A monobenzoate (MAPP-A/MAPP-B = 97.2/2.8, 1.00 g, 2.10 mmol.) obtained in Example 1 (3-4) was suspended in 1M aqueous sodium hydroxide solution (3 mL), and the solution was vigorously stirred for 10 minutes at room temperature after addition of toluene (3 mL). The organic solvent portion was then collected. After addition of 2M aqueous sodium hydroxide solution, the collected organic portion was dropwise added to a separately prepared toluene (0.3 mL) solution of ethyl chloroformate (0.34 mL, 3.13 mmol.) under chilling with ice. The mixture was stirred for one hour at room temperature, and the organic portion was collected. The organic portion was successively washed with water (1.5 mL), 2.5M aqueous hydrochloric acid (1 mL), water (1.5 mL) and saturated aqueous sodium chloride solution (3 mL), and then dried over anhydrous sodium sulfate. The solvent was distilled off under reduced pressure. The residue was dissolved in a methanol/water (4/1) mixture (10 mL). The resulting solution was heated under reflux for 15 hours after addition of potassium carbonate (0.44 g, 3.18 mmol.). After addition of water (5 mL), the reaction mixture was placed under reduced to distill methanol off. The residue was extracted with ethyl acetate. The extract was washed with saturated aqueous sodium chloride solution and dried over anhydrous sodium sulfate. After addition of 50% ethanol (2.6 mL) and fumaric acid (0.24 g, 2.07 mmol.), the solvent was distilled off under reduced pressure. The residue was stirred on an oil bath at 100°C (outer temperature) for 10 minutes, . The heater of the oil bath was switched off. The mixture was still stirred on the oil bath for gradual cooling. When the temperature of the oil bath reached 80°C, seed crystals (1 mg) were added. The mixture was further cooled overnight under stirring. The precipitated crystalline product was collected by filtration, washed with 50% ethanol (1 mL x 2), and dried overnight at 50°C under reduced pressure, to give 0.66 g (yield: 63.3%) of the desired compound as a white crystalline product.
mp: 164.6 - 165.6 °C

(C) MAPP-A monobenzoate (MAPP-A/MAPP-B = 97.2/2.8, 5.00 g, 10.5 mmol.) obtained in Example 1 (3-4) was suspended in 1M aqueous sodium hydroxide solution (15 mL), and extracted with toluene (15 mL, 5 mL). The toluene extract was washed with water (15 mL). The toluene extract was then vigorously stirred after addition of 3M aqueous sodium hydroxide solution (30 mL). To the stirred mixture was dropwisé added a toluene (10 mL) solution of trichloromethyl chloroformate (TCF: 1.90 mL, 15.8 mmol.) under chilling with ice so that the inner temperature of the mixture was kept at a temperature lower than 20°C. Subsequently, the mixture was further stirred for 30 minutes at room temperature, and the organic solvent portion was then collected. The collected organic portion was stirred for 30 minutes at room temperature after addition of an aqueous 10% ammonia solution (15 mL), and the organic solvent portion was collected. The collected portion was washed successively with water (15 mL) and saturated aqueous sodium chloride solution (15 mL), and placed under reduced pressure to distill the solvent off. After addition of 50% ethanol (15 mL) and fumaric acid (1.22 g, 10.5 mmol.), the residue was stirred on an oil bath at 100°C (outer temperature) for 10 minutes. The heater of the oil bath was switched off. The mixture was still stirred on the oil bath for gradual cooling.. When the temperature of the oil bath reached 80°C, seed crystals (1 mg) were added. The mixture was further cooled overnight under stirring. The precipitated crystalline product was collected by filtration, washed with 50% ethanol (5 mL x 2), and dried overnight overnight at 50°C under reduced pressure, to give 4.22 g (yield: 80.9%) of the desired product as a white crystalline product.
mp: 164.8 - 166.0 °C

### Process for preparing salt of MAPP-A with an organic acid

### [Example 3]

### (2RS) -1- [(1SR) -3-morpholino-1-phenylpropyl] amino-3-phenyl-2-propanol monobenzoate

A mixture of MAPP-A and MAPP-B (MAPP-A/MAPP-B = 52/48, 28.5 g, purity: 96%, 77.2 mmol.) and benzoic acid (9.43 g, 77.2 mmol.) were placed in isopropanol (379 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 190 mL of isopropanol, and dried under reduced pressure for 4 days at room temperature, to give 19.3 g of a white crystalline product. The obtained product was recrystallized from isopropanol (190 mL), and the recrystallized product was dried for 6 hours at room temperature, to give 15.4 g of a white crystalline product. The crystalline product was recrystallized from isopropanol (150 mL) and dried overnight at room temperature, to give 13.7 g of the desired compound as a white crystalline product. Yield: 36.5% from the MAPP-A,B mixture, and 70.2% from MAPP-A of the MAPP-A,B mixture.
mp: 137.0 - 140.0°C
MAPP-A/MAPP-B = 98.1/1.9

The determination of a ratio of diastereoisomer A/B for Examples 3 to 9 was performed by HPLC under the following conditions:
Column: GL SCIENCES INARTSIL ODS-3V (4.6×250 mm)
Moving phase: 2% NaH₂PO₄/CH₃CN = 10/3
Detection: UV 215 nm
Flow rate: 1.0 mL/min.

### [Example 4]

### (2RS) -1- [(1SR)-3-morpholino-1-phenylpropyl] amino-3-phenyl-2-propanol monobenzoate

A mixture of MAPP-A and MAPP-B (MAPP-A/MAPP-B = 50/50, 145 g, purity: 95%, 389 mmol.) and benzoic acid (50.0 g, 409 mmol.) were placed in isopropanol (1,950 mL). The resulting mixture was warmed until the mixture became homogeneous. The warmed solution was gradually cooled to room temperature under stirring. After the crystalline product was precipitated, the solution was allowed to stand overnight at room temperature. The precipitated crystalline product was collected by filtration, washed with 750 mL of isopropanol, and dried under reduced pressure 50°C for hours, to give 103.7 g (containing 3.0% isopropanol) of a white crystalline product. The obtained product (100 g) was recrystallized from isopropanol (970 mL), and the recrystallized product was dried at 50°C for 5 hours, to give 81.49 g (containing 2.3% isopropanol) of a white crystalline product. The crystalline product (80.0 g) was recrystallized from isopropanol (780 mL) and dried at 50°C for 6 hours and then at 30°C for 2 days, to give 72.03 g (containing 1.9% isopropanol) of the desired compound as a white crystalline product.
Yield: 39.8% from the MAPP-A,B mixture, and 79.6% from MAPP-A of the MAPP-A,B mixture.
mp: 138.0 - 140.8°C
MAPP-A/MAPP-B = 98.7/1.3

### Process for preparation of salt of (5RS)-5-benzyl-3-[(1SR)-3- morpholino-1-phenylpropyl]-1,3-oxazolidin-2-one with organic acid

### [Example 5]

### (5RS)-5-benzyl-3-[(1SR)-3-morpholino-1-phenyl-propyl]-1,3-oxazolidin-2-one fumarate

MAPP-A monobenzoate (MAPP-A/MAPP-B = 95.5/4.5, 1.35 g, 2.83 mmol.) was suspended in 1M aqueous sodium hydroxide solution (4 mL) and extracted with ethyl acetate (4 mL). The extract was washed with water (4 mL), dried over anhydrous sodium sulfate, and placed under reduced pressure to distill the solvent off. The residue was heated under reflux for 2.5 hours after addition of dimethyl carbonate (5 mL, 54.5 mmol.) and sodium methoxide (30 mg, 0.56 mmol.). Methanol and excessive dimethyl carbonate were distilled off under reduced pressure. The residue was washed with water and saturated aqueous sodium chloride solution after addition of ethyl acetate, dried over anhydrous sodium sulfate, and placed under reduced pressure to distill the solvent off, to give a crude product (1.10 g, purity: 83.3%, yield: 85.1%) of (5RS)-5-benzyl-3-[(1SR)-3-morpholino-1-phenylpropyl]-1,3-oxazolidin-2-one.

To the resulting oil were added 50% ethanol (3.8 mL) and fumaric acid (0.31 g, 2.67 mmol.). The mixture was stirred on an oil bath at 100°C (outer temperature) for 10 minutes. The heater of the oil bath was switched off. The mixture was still stirred on the oil bath for gradual cooling. When the temperature of the oil bath reached 60°C, seed crystals (1 mg) were added. The mixture was further cooled under stirring for 5 hours. The precipitated crystalline product was collected by filtration, washed with 50% ethanol, and air-dried overnight. The dried product was further dried under reduced pressure for 9 days at room temperature, to give 0.96 g (yield: 80.1%) of the desired product as a white crystalline product.
mp: 165.4 - 166.2 °C

### [Example 6]

### (5RS) -5-benzyl-3- [(1SR) -3-morpholino-1-phenylpropyl]-1,3-oxazolidin-2-one fumarate

MAPP-A monobenzoate (MAPP-A/MAPP-B = 98.7/1.3, 30.0 g, purity: 98.1%, 61.7 mmol.) was suspended in 1M aqueous sodium hydroxide solution (90 mL) and extracted with ethyl acetate. The extract was washed with water and saturated aqueous sodium chloride solution, dried over anhydrous sodium sulfate, and placed under reduced pressure to distill the solvent off. The residue was heated under reflux for 3.5 hours after addition of dimethyl carbonate (70 mL, 764 mmol.) and sodium methoxide (0.33 g, 6.11 mmol.). Methanol and excessive dimethyl carbonate were distilled off under reduced pressure. The residue was washed with water and saturated aqueous sodium chloride solution after addition of ethyl acetate, dried over anhydrous sodium sulfate, and placed under reduced pressure to distill the solvent off, to give a crude product (23.7 g, purity: 90.7%, yield: 91.6%) of (5RS)-5-benzyl-3-[(1SR)-3-morpholino-1-phenylpropyl]-1,3-oxazolidin-2-one.

To the resulting oil were added 50% ethanol (86 mL) and fumaric acid (7.09 g, 61.1 mmol.). The mixture was stirred on an oil bath at 100°C (outer temperature) for 10 minutes. The heater of the oil bath was switched off. The mixture was still stirred on the oil bath for gradual cooling. When the temperature of the oil bath reached 60°C, seed crystals (1 mg) were added. The mixture was further cooled overnight under stirring. The precipitated crystalline product was collected by filtration, washed with 50% ethanol, and dried overnight at 50°C under reduced pressure, to give 25.36 g (yield: 90.4%) of the desired compound as a white crystalline product.
mp: 166.2 - 167.2 °C

### [Example 7]

### (5RS)-5-benzyl-3-[(1SR)-3-morpholino-1-phenyl-propyl]-1,3-oxazolidin-2-one fumarate

MAPP-A monobenzoate (MAPP-A/MAPP-B = 95.5/4.5, 1.35 g, 2.83 mmol.) was suspended in a 1M aqueous sodium hydroxide solution (4 mL) and extracted with toluene (4 mL). The extract was washed with water, dried over anhydrous sodium sulfate, and placed under reduced pressure to distill the solvent off. The residue was heated under reflux for one hour in a vessel equipped with a Dean-Stark trap (40 mL volume, toluene (40 mL) was previously placed) after addition of dimethyl carbonate (1.7 mL, 14.1 mmol.) and toluene (15 mL).

To the reaction mixture was added sodium methoxide (15 mg, 0.28 mmol.), and the resulting mixture was heated under reflux for 2 hours. The mixture was then placed under reduced pressure to distill the solvent off, until the amount of the residue reached approx. 5 mL. The residue was washed with water and a saturated aqueous sodium chloride solution, and placed under reduced pressure to distill the solvent off, to give a crude product (1.11 g, purity: 85.0%, yield: 87.6%) of (5RS)-5-benzyl-3-[(1SR)-3-morpholino-1-phenylpropyl]-1,3-oxazolidin-2-one.

To the resulting oil were added 50% ethanol (3.9 mL) and fumaric acid (0.32 g, 2.76 mmol.). The mixture was stirred on an oil bath at 100°C (outer temperature) for 10 minutes. The heater of the oil bath was switched off. The mixture was still stirred on the oil bath for gradual cooling. When the temperature of the oil bath reached 60°C, seed crystals (1 mg) were added. The mixture was further cooled under stirring for 5 hours. The precipitated crystalline product was collected by filtration, washed with 50% ethanol, and air-dried overnight. The dried product was further dried under reduced pressure for 9 days at room temperature, to give 0.96 g (yield: 84.3%) of the desired compound as a white crystalline product.
mp: 165.6 - 170.0°C

### [Example 8]

### (5RS)-5-benzyl-3-[(1SR)-3-morpholino-1-phenyl-propyl]-1,3-oxazolidin-2-one fumarate

MAPP-A monobenzoate (MAPP-A/MAPP-B = 98.1/1.9, 1.35 g, 2.83 mmol.) was suspended in 1M aqueous sodium hydroxide solution (4 mL) and extracted with toluene (4 mL). The extract was washed with water, dried over anhydrous sodium sulfate (when the anhydrous sodium sulfate was removed, 3 mL of toluene was used), and heated under reflux for one hour in a vessel equipped with a Dean-Stark trap (40 mL volume, toluene (3 mL) was previously placed).

To the reaction mixture were added diphenyl carbonate (0.61 g, 2.85 mmol.) and sodium methoxide (15 mg, 0.28 mmol.). The resulting mixture was heated under reflux for 30 minutes. After addition of monoethanolamine (0.02 mL, 0.33 mmol.), the mixture was further heated under reflux for 30 minutes. The mixture was cooled to room temperature, washed with 2M aqueous sodium hydroxide solution, water, and saturated aqueous sodium chloride solution, dried over anhydrous sodium sulfate, and then placed under reduced pressure to distill the solvent off, to give a crude product (1.10 g, purity: 78.1%, yield: 79.8%) of (5RS)-5-benzyl-3-[(1SR)-3-morpholino-1-phenyl-propyl]-1,3-oxazolidin-2-one.

To the resulting oil were added 50% ethanol (2.9 mL) and fumaric acid (0.29 g, 2.50 mmol.). The mixture was stirred on an oil bath at 100°C (outer temperature) for 10 minutes. The heater of the oil bath was switched off. The mixture was still stirred on the oil bath for gradual cooling. When the temperature of the oil bath reached 60°C, seed crystals (1 mg) were added. The mixture was further cooled under stirring for 5 hours. The precipitated crystalline product was collected by filtration, washed with 50% ethanol, and dried under reduced pressure for 4 hours at room temperature, to give 0.99 g (yield: 86.4%) of the desired compound as a pinky white crystalline product.
mp: 165.2 - 166.6°C

### [Example 9]

### (5RS)-5-benzyl-3-[(1SR)-3-morpholino-1-phenyl-propyl]-1,3-oxazolidin-2-one fumarate

MAPP-A monobenzoate (MAPP-A/MAPP-B = 98.7/1.3, 30.0 g, purity: 98.1%, 61.7 mmol.) was suspended in 1M aqueous sodium hydroxide solution (90 mL) and extracted with toluene (90 mL). The extract was washed with water and saturated aqueous sodium chloride solution, dried over anhydrous sodium sulfate (when the anhydrous sodium sulfate was removed, 20 mL of toluene was used) for one hour, and heated under reflux in a vessel equipped with a Dean-Stark trap (40 mL volume, no solvent was previously placed therein), until 40 mL of toluene was trapped.

The Dean-Stark trap was removed, and in the reaction mixture were placed diphenyl carbonate (14.5 g, 67.7 mmol.) and sodium methoxide (0.33 g, 6.11 mmol.). The resulting mixture was heated under reflux for 3 hours. , After addition of monoethanolamine (0.74 mL, 12.4 mmol.), the mixture was further heated under reflux for 30 minutes. The mixture was cooled to room temperature, washed with 2M aqueous sodium hydroxide solution, water, and saturated aqueous sodium chloride solution, dried over anhydrous sodium sulfate, and then placed under reduced pressure to distill the solvent off, to give a crude product (23.6 g, purity: 95.3%, yield: 95.8%) of (5RS)-5-benzyl-3-[(1SR)-3-morpholino-1-phenylpropyl]-1,3-oxazolidin-2-one.

To the resulting oil were added 50% ethanol (86 mL) and fumaric acid (7.09 g, 61.1 mmol.). The mixture was stirred on an oil bath at 100°C (outer temperature) for 10 minutes. The heater of the oil bath was switched off. The mixture was still stirred on the oil bath for gradual cooling. When the temperature of the oil bath reached 60°C, seed crystals (1 mg) were added. The mixture was further cooled overnight under stirring. The precipitated crystalline product was collected by filtration, washed with 50% ethanol, and dried under reduced pressure overnight at 50°C, to give 26.58 g (yield: 90.6%) of the desired product as a pinky white crystalline product.
mp: 166.4 - 167.0°C

### Preparation of olefinic compound from MAPP-B

### [Reference Example 8]

### 1-(3-Morpholino-1-phenylpropyl]amino-3-phenyl-2-propene (olefinic compound)

MAPP-B-excessive benzoate (A/B = 27.1/72.9, 13.4 g, 1 mmol) was suspended in 1M aqueous sodium hydroxide solution (4 mL), and extracted with ethyl acetate. The extract was washed with water, dried over anhydrous sodium sulfate, and placed under reduced pressure to distill the solvent off. After addition of diethyl carbonate (45 mL) and sodium methoxide (1.52 g, 28.1 mmol.), the residue was heated under reflux for 4 hours. In the course of reflux, the condenser was removed to evaporate methanol and ethanol. The mixture was further heated under reflux for 4 hours. After addition of sodium methoxide (0.76 g, 14.1 mmol), the reaction mixture was furthermore heated under reflux 4 hours. The insolubles were filtered off, and the filtrate was concentrated under reduced pressure. The residue was, after ethyl acetate was added, washed with water and saturated aqueous sodium chloride solution, dried over anhydrous sodium sulfate, and placed under reduced pressure, to give 8.76 g (yield: 92.6%) of the desired product (trans-compound) as a pale orange oil.
¹H NMR (CDCl₃, 400 MHz) δ: 1.75-2.04 (2H, m), 2.20-2.46 (7H, m), 3.15-3.28 (2H, m), 3.67-3.69 (4H, m), 3.76 (1H, t, J=7Hz), 6.25 (1H, dt, J=7Hz, 16Hz), 6.45 (1H, d, J=16Hz), 7.15-7.36 (10H, m).

### Preparation of MAPP-A/B mixture from olefinic compound

### [Reference Example 9]

### 1-(3-Morpholino-1-phenylpropyl]amino-3-phenyl-2-propanol (mixture of MAPP-A and MAPP-B)

### (9-1)

The olefin compound (336.5 mg, 1.00 mmol.) of Reference Example 8 was dissolved in anhydrous THF (1 mL) in a nitrogen gas atmosphere. The solution was chilled with ice, and stirred for one hour at room temperature after addition of BH₃·THF/THF (0.98 M, 4.1 mL, 4.02 mmol). The reaction mixture was again chilled with ice, and stirred for one hour at room temperature after addition of water (0.5 mL), 4M aqueous sodium hydroxide solution (3.4 mL) and 35% aqueous hydrogen peroxide solution (0.26 mL). The reaction solution was, after addition of 10 wt.% aqueous sodium hydrogen sulfite solution (1 mL), extracted with ethyl acetate. The extract was placed under reduced pressure to distill the solvent off. To the residue was added 6M aqueous hydrochloric acid. (2 mL) under chilling with ice. The mixture was stirred at 100°C for 30 minutes. Under chilling with ice, 4M aqueous sodium hydroxide solution was added to the resulting mixture until the mixture became to show pH 14. The mixture was extracted with ethyl acetate. The extract was dried over anhydrous sodium sulfate and placed under reduced pressure to distill the solvent off. From the residue was separated a 63 mg portion: The separated portion was purified by collective TLC (35% aq. NH₃/ methanol/benzene = 1/10/50), to obtain 41 mg (yield: 69%) of the desired product as pale yellow oil, containing 3% of 1-propanol product.
¹H NMR (CD₃OD, 400 MHz) δ: 1.70-2.75 (12H, m), 3.60-3.70 (5H, m), 3.80-3.95 (1H, m), 7.10-7.35 (10H, m).

### (9-2)

The olefin compound (6.72 g, 20.0 mmol.) of Reference Example 8 was dissolved in anhydrous THF (20 mL) in a nitrogen gas atmosphere. The solution was chilled with ice, and stirred for 2.5 hours at room temperature after dropwise addition of BH₃·THF/THF (0.98 M, 81.7 mL, 80.1 mmol) for a period of longer than 30 minutes. The reaction mixture was further stirred for 2.5 hours at room temperature. Under chilling with ice, to the reaction mixture were added water (10 mL) for a period of longer than 20 minutes, and further 4M aqueous sodium hydroxide solution (68 mL, for a period of longer than 10 minutes). The mixture was then stirred for 2 hours at room temperature after addition of 35% aqueous hydrogen peroxide solution (5.2 mL). The mixture was further stirred for 30 minutes at room temperature after addition of 10% aqueous sodium hydrogen sulfite solution (20 mL), and extracted with ethyl acetate. The extract was placed under reduced pressure to distill the solvent off. The residue was heated under stirring at 100°C for 15 minutes after 6M aqueous hydrochloric acid (40 mL) was added under chilling with ice. The mixture was made to show pH 14, by addition of 4M aqueous sodium hydroxide solution under chilling with ice, and extracted with ethyl acetate. The ethyl acetate portion was washed with saturated aqueous sodium chloride solution, dried over anhydrous sodium sulfate, and placed under reduced pressure to distill the solvent off, to give 7.62 g of a crude product of MAPP as pale yellow oil.

A portion of the crude product (340 mg) was dissolved in toluene and extracted with 1M aqueous acetic acid. The aqueous portion was washed with toluene, and then extracted with toluene after addition of 1M aqueous sodium hydroxide solution. These procedures were further repeated twice. The resulting toluene solution was washed with saturated aqueous sodium chloride solution, dried over sodium sulfate, and placed under reduced pressure to distill the solvent off, to obtain 209 mg of a mixture of MAPP-A and MAPP-B as colorless oil.

### (9-3)

The olefin compound (336.5 mg, 1.00 mmol.) of Reference Example 8 was dissolved in anhydrous THF (1 mL) in a nitrogen gas atmosphere. The solution was chilled with ice, and stirred for 2 hours at room temperature after addition of BH₃·SMe₂/THF (2M, 3 mL, 1.5 mmol). The reaction mixture was again chilled with ice, and stirred for one hour at room temperature after addition of water (0.5 mL), 4M aqueous sodium hydroxide solution (3.4 mL) and 35% aqueous hydrogen peroxide solution (0.26 mL). The reaction solution was, after addition of 10 wt.% aqueous sodium hydrogen sulfite solution (1 mL), extracted with ethyl acetate. The extract was placed under reduced pressure to distill the solvent off. To the residue was added 6M aqueous hydrochloric acid (2 mL) under chilling with ice. The mixture was stirred at 100°C for 30 minutes. Under chilling with ice, 4M aqueous sodium hydroxide solution was added to the resulting mixture until the mixture became to show pH 14. The mixture was extracted with ethyl acetate. The extract was dried over anhydrous sodium sulfate and placed under reduced pressure to distill the solvent off. From the residue was separated a 63 mg portion. The separated portion was purified by collective TLC (35% aq. NH₃/methanol/benzene = 1/10/50) to obtain 30 mg (yield: 54%) of the desired product as pale yellow oil, containing 13% of 1-propanol product.

### (9-4)

In a nitrogen gas atmosphere, NaBH₄ (57 mg, 1.51 mmol.) was suspended in anhydrous THF (3 mL). The suspension was stirred at 0°C for 30 minutes, after BF₃·EtO (0.25 mL, 2.03 mmol.) was added for a period of 30 minutes. To the mixture was added a solution of the olefin compound of Reference example 8 (169 mg, 0.50 mmol.) in anhydrous THF (3 mL) under chilling with ice. The mixture was stirred for 3 hours at room temperature. Subsequently, to the mixture were added successively water (0.25 mL, for 5 min.) and 4M aqueous sodium hydroxide solution (1.7 mL, for 5 min.). The resulting mixture was, after addition of 35% aqueous sodium hydrogen peroxide solution (0.13 mL), stirred for 2 hours at room tem-perature. The mixture was then extracted with ethyl acetate after addition of 10% aqueous sodium hydrogen sulfite solution (0.5 mL). The extract was placed under reduced pressure to distill the solvent off. The residue was heated under stirring at 100°C for 15 minutes, after 6M aqueous hydrochloric acid (1 mL) was added under chilling with ice. The mixture was made to show pH 14, by addition of 4M aqueous sodium hydroxide solution under chilling with ice, and extracted with ethyl acetate. The ethyl acetate portion was washed with saturated aqueous sodium chloride solution, dried over anhydrous sodium sulfate, and placed under reduced pressure to distill the solvent off, to give 169 mg of a crude product of a mixture of MAPP-A and MAPP-B as colorless oil.

## Claims

1. A process for preparing (5RS)-5-benzyl-3-[(1SR)-3-morpholino-1-phenylpropyl]-1,3-oxazolidin-2-one which comprises the steps of:
reacting (2RS)-1-[(TSR)-3-morpholino-1-phenyl-propyl] amino-3-phenyl-2-propanol with a compound having the formula (I):
R₂C=O (I)
wherein R represents a chlorine atom, an alkoxy group, an aryloxy group or an amino group,
or a chloroformic acid ester; and
cyclizing the resulting product.

2. A process for preparing (5RS)-5-benzyl-3-[(1SR)-3-morpholino-1-phenylpropyl]-1,3-phenylpropyl]-2-one which comprises the steps of:
dissolving an acid and a mixture of (2RS)-1-[(1SR)-3-morpholino-1-phenylpropyl]amino-3-phenyl-2-propanol and (2RS) -1- [(1RS)-3-morpholino-1-phenylpropyl] amino-3-phenyl-2-propanol in a solvent to obtain a salt of the former (1SR)-compound utilizing difference of solubility between the salt of (1SR) -compound and a salt of the latter (1RS)-compound;
bringing a basic compound into contact with the salt of (1SR)-compound to produce a free (1SR)-compound;
reacting thus produced (2RS)-1-[(1SR)-3-morpholino-1-phenylpropyl] amino-3-phenyl-2-propanol with a compound having the formula (I):
R₂C=O (I)
wherein R represents a chlorine atom, an alkoxy group, an aryloxy group or an amino group,
or a chloroformic acid ester; and
cyclizing the resulting product.

3. The process of claim 2, wherein the acid is benzoic acid, succinic acid or p-toluenesulfonic acid.
